(19) Europäisches Patentamt
European Patent Office
Office européen des brevets

(11) Veröffentlichungsnummer: **0 245 730 A1**

(12) # EUROPÄISCHE PATENTANMELDUNG

(21) Anmeldenummer: **87106380.6**

(51) Int. Cl.⁴: **C07C 147/06** , C08G 75/20

(22) Anmeldetag: **30.04.87**

(30) Priorität: **13.05.86 DE 3616063**

(43) Veröffentlichungstag der Anmeldung: **19.11.87 Patentblatt 87/47**

(84) Benannte Vertragsstaaten: **DE FR GB IT NL**

(71) Anmelder: **BAYER AG**
**Konzernverwaltung RP Patentabteilung**
**D-5090 Leverkusen 1 Bayerwerk(DE)**

(72) Erfinder: **Reuter, Knud, Dr.**
**Scheiblerstrasse 99**
**D-4150 Krefeld(DE)**

(54) **Verfahren zur Herstellung aromatischer (Poly)Sulfone.**

(57) Verfahren zur Herstellung von gegebenenfalls polymeren Arysulfonen, in dem Arylsulfinsäuren der Formel (I) oder ihre Salze

$$Ar^1 \left[ SO_2M \right]_m \qquad (I),$$

in welcher

$Ar^1$ für gegebenenfalls mit $C_1$-$C_4$-Alkyl, oder Halogen wie Cl, Br oder $NO_2$ substituiertes $C_6$-$C_{14}$-Aryl ($m = 1$), oder $C_6$-$C_{14}$-Arylen ($m = 2$) steht,

m für die Zahl 1 oder 2 steht und

M für Wasserstoff oder Alkali wie Li, Na, K und die $NR_4$-Gruppe steht, in welcher R für $C_2$-$C_4$-Alkyl steht,

mit aromatischen Nitroverbindungen der Formel (II)

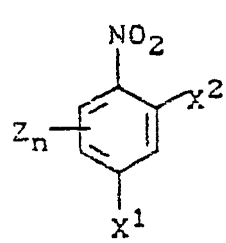

(II),

in welcher

$X^1$ H oder $-SO_2-R^2$ und

$X^2$ H oder $-SO_2-R^2$ ist und wobei

$X^1$ und $X^2$ nicht gleichzeitig H bzw. $-SO_2-R^2$ sein dürfen,

EP 0 245 730 A1

Xerox Copy Centre

$R^2$ für einen gegebenenfalls mit $C_1$-$C_4$ Alkyl, Cl, Br substituierten $C_6$-$C_{14}$ Arylrest,

Z für $C_1$-$C_4$-Alkyl, $C_6$-$C_{10}$-Aryl, $C_1$-$C_4$-Alkoxy, $C_6$-$C_{10}$-Aryloxy, $C_1$-$C_4$-Dialkylamino, $C_6$-$C_{10}$-Aryl-$C_1$-$C_4$-alkylamino, $C_6$-$C_{10}$-diarylamino steht,

n für die Zahl 0,1,2,3 oder 4 steht,

umgesetzt werden.

## Verfahren zur Herstellung aromatischer (Poly)Sulfone

Die vorliegende Erfindung betrifft ein Verfahren zur Herstellung aromatischer (Poly)Sulfone.

Zur Herstellung aromatischer Sulfone ist eine Reihe von Verfahren bekannt, wie sie zum Beispiel in Ullmann's Encyclopädie der Technischen Chemie, 4. Auflage, Bd. 22, S. 323 ff., beschrieben sind, z.B. Oxidation von Sulfiden bzw. Sulfoxiden, Sulfonierung aromatischer Kohlenwasserstoffe, Umlagerung von Sulfonsäureestern zu Hydroxydiphenylsulfonen, die Addition von Sulfinsäuren an Chinone und Chinonimine sowie die nucleophile Substitution von Chlor in o-oder p-Chlornitrobenzolen.

Zur Herstellung von Sulfonen aus Nitroaromaten durch Substitution einer $NO_2$-Funktion sind bisher nur einige spezielle Beispiele bekannt, z.B. Synthese von Poly(arylsulfonyl)benzolen durch Umsetzung von Chlornitrobenzolen mit Arylsulfinaten (J. Chem. Soc. 1936 , 216; 1937, 246 und 1939, 902).

Eine $NO_2$-Gruppe kann nur dann gegen Sulfinat in guten Ausbeuten ausgetauscht werden, wenn entweder eine Nitrogruppe in o-Position oder zwei Tolylsulfonylgruppen (o-und p-Position) für eine hinreichende Aktivierung sorgen.

Daß eine zweite Nitrogruppe in o-oder p-Stellung ausreicht, um den Austausch eines $NO_2$ gegen Sulfinat zu ermöglichen, ist bekannt (J. Org. Chem. 41 (9), 1560 (1976) (C.A. 87 565 (1977)).

Die vorliegende Erfindung beschreibt ein Verfahren zur Herstellung von Sulfonen und Polysulfonen durch nucleophile aromatische Substitution von Nitrogruppen, bei dem die Nitroaromaten weder durch eine zusätzliche $NO_2$-Gruppe in o-oder p-Stellung noch durch 2 oder mehr o-oder p-ständige Arylsulfonylgruppen aktiviert werden.

Gegenstand der Erfindung ist somit ein Verfahren zur Herstellung von gegebenenfalls polymeren Arylsulfonen, das dadurch gekennzeichnet ist, daß Arylsulfinsäuren der Formel (I) oder ihre Salze

$$Ar^1\left[\, SO_2M \,\right]_m \qquad (I),$$

in welcher

$Ar^1$ für gegebenenfalls mit $C_1$-$C_4$-Alkyl, oder Halogen wie Cl, Br oder $NO_2$ substituiertes $C_6$-$C_{14}$-Aryl (m = 1), oder $C_6$-$C_{14}$-Arylen (m = 2) steht,

m für die Zahl 1 oder 2 steht und

M für Wasserstoff oder Alkali wie Li, Na, K und die $NR_4$-Gruppe steht, in welcher R für $C_1$-$C_4$-Alkyl steht,

mit aromarischen Nitroverbindungen der Formel (II)

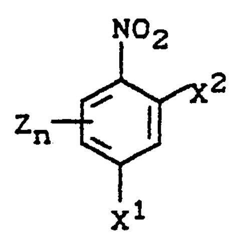

(II),

in welcher

$X^1$ H oder -$SO_2$-$R^2$ und

$X^2$ H oder -$SO_2$-$R^2$ ist und wobei

$X^1$ und $X^2$ nicht gleichzeitig H bzw. -$SO_2$-$R^2$ sein dürfen,

$R^2$ für einen gegebenenfalls mit $C_1$-$C_4$-Alkyl, Cl, Br substituierten $C_6$-$C_{14}$ Arylrest,

Z für $C_1$-$C_4$-Alkyl, $C_6$-$C_{10}$-Aryl, $C_1$-$C_4$-Alkoxy, $C_6$-$C_{10}$-Aryloxy, $C_1$-$C_4$-Dialkylamino, $C_6$-$C_{10}$-Aryl-$C_1$-$C_4$-alkylamino, $C_6$-$C_{10}$-Diarylamino steht

und

n für die Zahl 0,1,2,3 oder 4 steht,

umgesetzt werden.

Vorzugsweise werden im erfindungsgemäßen Verfahren (Poly) Arylsulfone aus Arylsulfinsäuren oder ihren Salzen mit Nitrosulfonen der Formel (III) hergestellt

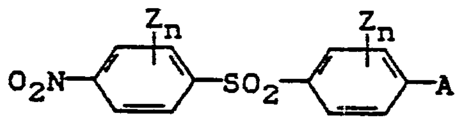

(III),

in welcher

$Z_n$ die bei Formel (II) angegebene Bedeutung hat,

A ein Wasserstoffatom, ein Halogen wie F, Cl, Br, J oder eine Nitrogruppe ist,

und wobei für den Fall, daß A für Halogen oder eine Nitrogruppe steht, A ebenfalls mit der Arylsulfinsäure bzw. ihrem Salz umgesetzt werden kann.

In einer besonderen Ausführungsform des Verfahrens wird das Nitrosulfon in situ aus dem Chlornitrobenzol gebildet, z.B. entsprechend der Gleichung

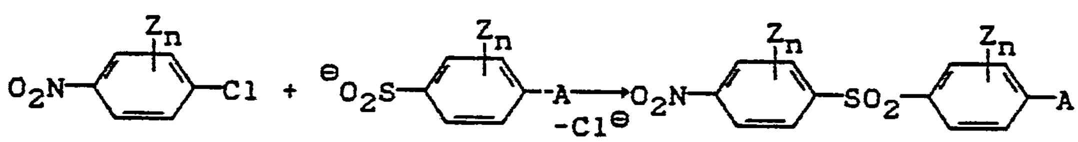

(IV),

wobei

$Z_n$ und A die bei Formel (III) angegebene Bedeutung haben.

Vorzugsweise wird diese besondere Ausführungsform des Verfahrens so durchgeführt, daß aus einem Nitrosulfon der Formel (IV)

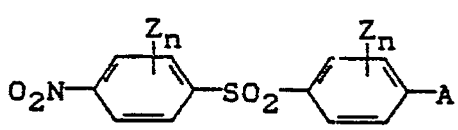

(IV),

in welcher Z, A und n die bei Formel (II) angegebene Bedeutung haben,

oder einem Chlornitrobenzol der Formel (V)

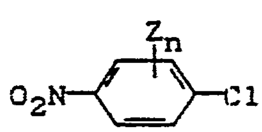

(V),

in welcher Z und n die bei Formel (II) angegebene Bedeutung haben,

durch Umsetzung mit der Arylsulfinsäure (bzw. ihrem Salz) der Formel (VI)

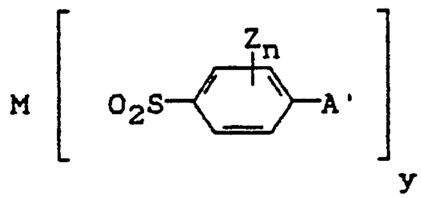
(VI),

in welcher

M die in Formel (I) angegebene Bedeutung hat,

Z und n die bei Formel (II) angegebene Bedeutung haben

und

A′ für Halogen wie Cl, Br, J oder eine Nitrogruppe steht,
y für die Zahl 1 oder 2 und für den Fall, daß y für die Zahl 1 steht,
M Wasserstoff oder ein Alkaliion wie Li, Na, K bedeutet, und für den Fall, daß y für die Zahl 2 steht, M für Erdalkali wie Mg, Ca oder Zn steht,

(Poly)sulfone aufgebaut werden.

Ein weiteres, besonderes Verfahren zur Herstellung von aromatischen (Poly)Sulfonen ist dadurch gekennzeichnet, daß mit Nitroverbindungen der Formel (II)

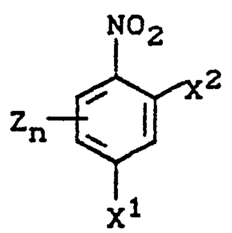
(II),

aromatische Disulfinsäuren (bzw. deren Salze) der Formeln (VII),

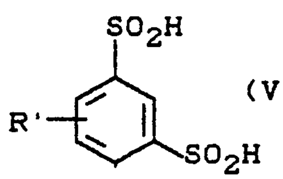
(VIIa)
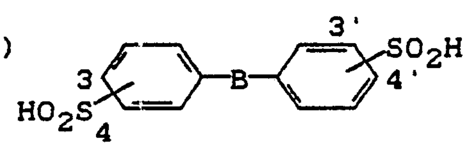
(VIIb)

umgesetzt werden, wobei

R′ = H, $C_{1-18}$-Alkyl,

B = O oder $SO_2$ ist und

$X^1$, $X^2$, Z und n die bei Formel (II) angegebene Bedeutung haben sollen.

In einer weiteren Variante des Verfahrens zur Herstellung von aromatischen (Poly)Sulfonen werden Nitroverbindungen der Formel (II)

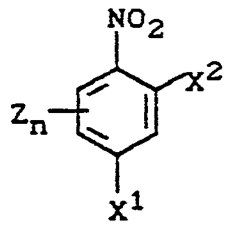

(II),

mit Sulfinsäuren der Formel (VIII)

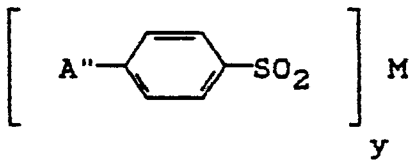

(VIII),

in welcher A" für H, $CH_3$, Halogen wie Cl, oder $NO_2$ steht und M und y die bei Formel (VI) angegebene Bedeutung haben, umgesetzt.

Bevorzugt werden nach dem erfindungsgemäßen Verfahren zur Herstellung von (Poly)Sulfonen die Lithium-, Natrium-, Kalium-oder Ammoniumsalze der Sulfinsäuren oder deren Salze mit organischen Basen, z.B. Tetramethylammonium, Benzyltrimethylammonim etc. eingesetzt.

Besonders bevorzugt wird im erfindungsgemäßen Verfahren die Umsetzung in einem dipolaren aprotischen Lösungsmittel, wie Dimethylsulfoxid (DMSO) oder Dimethylformamid, ganz besonders bevorzugt in DMSO, durchgeführt, oder in Lösungsmittelgemischen, die überwiegend aus dipolaren aprotischen Lösungsmitteln, bevorzugt DMSO, bestehen.

Erfindungsgemäß können als Arylsulfinsäuren z.B. Benzolsulfinsäure, p-Toluolsulfinsäure, p-Chlorbenzolsulfinsäure, 4-Nitorbenzolsulfinsäure, Benzol-1,3-disulfinsäure, Toluol-2,4-disulfinsäure, Diphenylether-4 ,4'-disulfinsäure, Diphenylsulfon-3,3'-disulfinsäure eingesetzt werden, die z.B. mit 4,4'-Dinitrodiphenylsulfon, 4-Nitro-4'-chlordiphenylsulfon, 4-Nitro-4'-fluordiphenylsulfon umgesetzt werden können.

Die nach dem erfindungsgemäßen Verfahren hergestellten Produkte eignen sich z.B. als Rohstoffe für Kunststoffe, beispielsweise derart, daß die hochmolekularen Produkte zu thermoplastischen Werkstoffen verarbeitet werden, oder derart, daß neidermolekulare Produkte, welche geeignete funktionelle Gruppen tragen (z.B. Cl, $NO_2$, $NH_2$, Carboxyl usw.) mit dazu passenden Reaktionspartnem zu hochmolekularen Produkten umgesetzt und diese anschließend zu thermoplastischen Werkstoffen verarbeitet werden.

## Beispiele

### Beispiel 1

21,94 g Natrium-benzolsulfinat (70,2 %ig) und 15,4 g 4,4'-Dinitrodiphenylsulfon (Molverh. 2:1) werden in 100 ml wasserfr. DMSO 24 h auf 150°C erhitzt. Nach Abkühlen auf Raumtemperatur können 5 g eines Rohproduktes abfiltriert werden, die aus Chlorbenzol umkristallisiert werden können. Schmelzpunkt nach Umkristallisierung 320-326°C; nach Massenspektrum und Elementaranalyse kann das Produkt mit der Formel (IX) beschrieben werden:

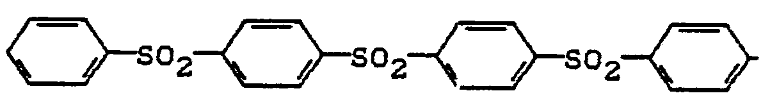

(IX)

Beispiel 2

21,9 g Natrium-p-toluolsulfinat (76,6 %ig) und 15,4 g 4,4'-Dinitrodiphenylsulfon (Molverh. 2:1) werden in 100 ml wasserfreiem DMSO 24 h auf 150°C erhitzt. Nach Abkühlen auf Raumtemperatur können 7,5 g eines Rohproduktes abfiltriert werden, die aus Chlorbenzol umkristallisiert werden können. Schmelzpunkt nach Umkristallisation 327°C; nach Massenspektrum und Elementaranalyse kann das Produkt mit der Formel (X) beschrieben werden

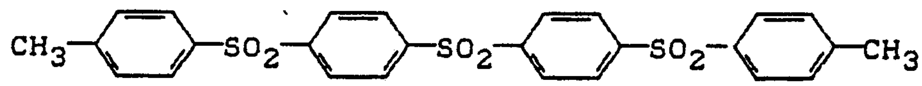 (X)

Beispiel 3

43,4 g Natrium-p-toluolsulfinat und 14,2 g p-Chlornitrobenzol (Molverh. 2,2:1) werden in 300 ml wasserfreiem DMSO 13 h bei 140°C umgesetzt. Das bei Abkühlen auf Raumtemperatur ausgefallene Rohprodukt (22,6 g) wird 1 h mit Wasser aufgekocht und aus Chlorbenzol umkristallisiert, Schmelzpunkt nach der Umkrist.: 247-250°C. Nach IR-und NMR-Spektrum sowie Elementaranalyse kann das Produkt mit der Formel (XI) beschrieben werden:

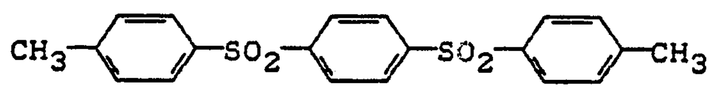 (XI)

Beispiel 4

178,7 g Natrium-p-chlorbenzolsulfinat und 70,9 g p-Chlornitrobenzol (Molverh. 2:1) werden in 750 ml wasserfreiem DMSO 17 h auf 140°C erhitzt. Das beim Abkühlen auf Raumtemperatur ausgefallene Produkt besteht nach NMR-Spektrum und Elementaranalyse aus den folgenden Verbindungen im Gewichtsverhältnis von ca. 4:1, die durch die Formeln (XII) und (XIII) beschrieben werden können.

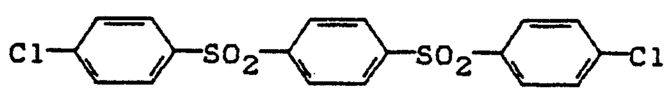 (XII)

(erfindungsgemäß)

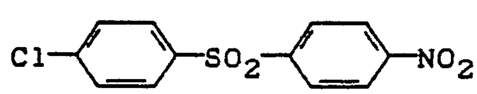 (XII)

(nicht Gegenstand der Erfindung)

Nach Umkristallisation aus Chlorbenzol schmilzt das Produkt bei 274-288°C (Zusammensetzung nach Elementaranalyse unverändert).

Beispiel 5

27,5 g Natrium-benzol-1,3-disulfinat (etwa 90 %ig) und 15,75 g p-Chlornitrobenzol werden in 200 ml wasserfreiem DMSO 24 h auf 150°C erhitzt. Nach Filtration wird aus der Mutterlauge mit Methanol ein Polymer gefällt, das entsprechend der Struktur der Reaktionspartner und nach der Elementaranalyse durch Formel (XIV) beschrieben werden kann:

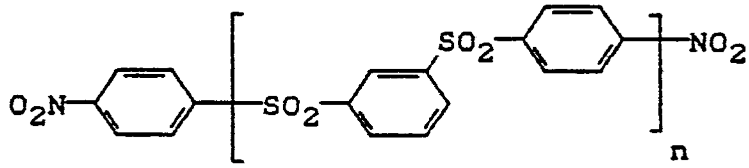

n ~ 22
$M_n$ ~ 6500

(XIV)

## Ansprüche

1. Verfahren zur Herstellung von gegebenenfalls polymeren Arysulfonen, das dadurch gekennzeichnet ist, daß Arylsulfinsäuren der Formel (I) oder ihre Salze

$$Ar^1\left[SO_2M\right]_m \qquad (I),$$

in welcher

$Ar^1$ für gegebenenfalls mit $C_1$-$C_4$-Alkyl, oder Halogen wie Cl, Br oder $NO_2$ substituiertes $C_6$-$C_{14}$-Aryl (m = 1), oder $C_6$-$C_{14}$-Arylen (m = 2) steht,

m für die Zahl 1 oder 2 steht und

M für Wasserstoff oder Alkali wie Li, Na, K und die $NR_4$-Gruppe steht, in welcher R für $C_2$-$C_4$-Alkyl steht,

mit aromatischen Nitroverbindungen der Formel (II)

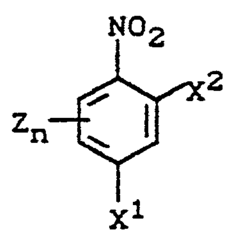

(II),

in welcher

$X^1$ H oder $-SO_2-R^2$ und

$X^2$ H oder $-SO_2-R^2$ ist und wobei

$X^1$ und $X^2$ nicht gleichzeitig H bzw. $-SO_2-R^2$ sein dürfen,

$R^2$ für einen gegebenenfalls mit $C_1$-$C_4$ Alkyl, Cl, Br substituierten $C_6$-$C_{14}$ Arylrest,

Z für $C_1$-$C_4$-Alkyl, $C_6$-$C_{10}$-Aryl, $C_1$-$C_4$-Alkoxy, $C_6$-$C_{10}$-Aryloxy, $C_1$-$C_4$-Dialkylamino, $C_6$-$C_{10}$-Aryl-$C_1$-$C_4$-alkylamino, $C_6$-$C_{10}$-diarylamino steht,

n für die Zahl 0,1,2,3 oder 4 steht,

umgesetzt werden.

2. Verfahren nach Anspruch 1, dadurch gekennzeichnet, daß (Poly)Arylsulfone aus Arylsulfinsäuren oder ihren Salzen mit Nitrosulfonen der Formel (III) hergestellt werden:

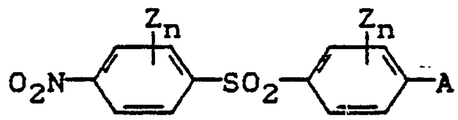

(III),

in welcher

$Z_n$ die bei Formel (II) angegebene Bedeutung hat,

A ein Wasserstoffatom, ein Halogen wie F, Cl, Br, J oder eine Nitrogruppe ist,
und für den Fall, daß A für Halogen oder eine Nitrogruppe steht, A ebenfalls mit der Arylsulfinsäure bzw. ihrem Salz umgesetzt werden kann.

3. Verfahren nach Anspruch 1, dadurch gekennzeichent, daß das Nitrosulfon in situ aus dem Chlornitrobenzol gebildet wird.

Europäisches Patentamt

# EUROPÄISCHER RECHERCHENBERICHT

Nummer der Anmeldung

EP 87 10 6380

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DE-A-1 806 993 (BAYER)<br>* Beispiel *<br>--- | 1-2 | C 07 C 147/06<br>C 08 G 75/20 |
| A | FR-A-2 317 285 (I.C.I.)<br>--- | | |
| A | JOURNAL OF THE CHEMICAL SOCIETY, CHEMICAL COMMUNICATIONS, Seiten 523-524, 1985, Letchworth, GB; N. ONO et al.: "Direct conversion of allylic nitro compounds into allyl sulphides and allyl sulphones"<br>--- | | |
| A,D | JOURNAL OF ORGANIC CHEMISTRY, Band 41, Nr. 9, 1976, Seiten 1560-1564, Easton, US; N. KORNBLUM et al.: "Displacement of the nitro group of substituted nitrobenzenes - a synthetically useful process"<br>* Tabelle I *<br>----- | | RECHERCHIERTE SACHGEBIETE (Int. Cl.4)<br>C 07 C 147/00<br>C 08 G 75/00 |

Der vorliegende Recherchenbericht wurde für alle Patentansprüche erstellt.

| Recherchenort | Abschlußdatum der Recherche | Prüfer |
|---|---|---|
| DEN HAAG | 17-07-1987 | VAN GEYT J.J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82